(19) Europäisches Patentamt
European Patent Office
Office européen des brevets

(11) **EP 4 785 995 A1**

(12) **EUROPEAN PATENT APPLICATION**

(43) Date of publication:
**05.08.2026** Bulletin 2026/32

(21) Application number: 25155478.8

(22) Date of filing: **03.02.2025**

(51) International Patent Classification (IPC):
***A61N 5/10*** *(2006.01)*

(52) Cooperative Patent Classification (CPC):
**A61N 5/1071;** A61N 5/1001; A61N 2005/1021

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **RaySearch Laboratories AB**
**104 30 Stockholm (SE)**

(72) Inventors:
• **Angerud, Agnes**
**175 69 Järfälla (SE)**
• **Kähler, Felix**
**11639 Stockholm (SE)**

(54) **METHOD, COMPUTER PROGRAM PRODUCT, AND SYSTEM FOR PERFORMING DOSIMETRY**

(57) A method of performing dosimetry for radiopharmaceutical therapy involving the distribution of a radionuclide to a patient, comprising
- Determining activity in a volume of interest based on the one or more nuclear images, the activity being representative of the number of radioactive decays as a function of time,
- Calculating an absorbed dose based on the time-integrated activity and an uncertainty in the absorbed dose based on the uncertainty for the total activity

Fig. 1

## Description

Technical Field

The present invention relates to a method of performing dosimetry, in particular in radiopharmaceutical therapy, a computer program product and a system for performing the method.

Background

Radiopharmaceutical therapy, also referred to as radionuclide therapy, involves the use of drugs, known as radiopharmaceuticals or radionuclides, for delivering radiation dose directly to a target in a patient. The radionuclides are adapted to localize to specific locations within the body to deliver their dose locally. This means that the delivery can be very precisely aimed at the sick tissue, causing fewer side effects.

One challenge of radiopharmaceutical treatment is dosimetry, that is, the measuring or estimation of the dose delivered to a patient. Depending on the interpretation of the European council directive 2013/59/EURATOM, dosimetry is required for a personalized optimization of the treatment for each patient. Dose estimation for radiopharmaceutical therapy involves larger inherent uncertainties compared to external beam radiation treatment (EBRT). Factors contributing to these uncertainties include calibration of the imaging apparatus to the radiation source for SPECT images and generally the conversion from counts to the appropriate unit (for example, activity), delineation of volumes of interest, where smaller volumes have larger uncertainties, determination of the activity as a function of time, time-integration of the accumulated activity, and the dose computation itself. Such time-integration may include models for biological effects, for example, based on the LQ model. The term activity in the context of this disclosure is defined as the number of radioactive decays per time unit. Activity is typically measured for one or more regions of interest, such as organs at risk (OAR) and/or lesions. Often, the activity accumulated in the regions of interest over a treatment fraction are considered. Image uncertainties can depend on, for example, position in the image, patient geometry, image protocol such as reconstruction settings, and motion during imaging. Because of these uncertainties, dosimetry is not performed systematically for radiopharmaceutical therapy in many clinics.

When planning a radiotherapy treatment, it is desirable to take uncertainties into account, to ensure that the treatment will be effective and that the resulting dose to the patient will be within acceptable limits.

One way of reducing the uncertainties involves the use of a number of images taken at different points in time, to determine the variation in the level of activity over time. The images are typically PET or SPECT scans, or PET/CT or SPECT/CT scans, but may also be other types of images, such as planar scintigraphy. 2D images may also be used. For example, a PET or SPECT scan taken at a point in time provides an indication of the radiation activity at that point in time in different areas of the scan. With a number of scans taken at different time points, multiple timepoint dosimetry therefore enables the monitoring of the activity of the radiopharmaceutical over time.

One disadvantage of the multiple timepoint method is that obtaining a scan takes time and causes discomfort to a patient. For example, obtaining a PET scan takes a long time, typically around 20 minutes, which means that it is not feasible to obtain several PET scans of each patient at different points in time for dosimetry purposes because the workload involved places high requirements on equipment and personnel. Multiple time point imaging also puts a high demand on patients, who are often not in a situation where multiple imaging sessions are comfortable or feasible.

Jackson et al.: Radiation Dosimetry in 177Lu-PSMA-617 Therapy Using a Single Posttreatment SPECT/CT Scan: A Novel Methodology to Generate Time- and Tissue-Specific Dose Factors, The Journal of Nuclear Medicine, Vol. 61, No. 7, July 2020, referred to in the following as Jackson et al., discloses a method enabling single time-point dosimetry by obtaining cohort data for the relevant treatment scheme from a group of patients. The cohort data are processed to generate a population pharmacokinetic model of the activity as a function of time. This can be used in patient treatment to determine a dose distribution.

Hänscheid et al.: Dose Mapping After Endoradiotherapy with 177Lu-DOTATATE/DOTATOC by a Single Measurement After 4 Days, The Journal of Nuclear Medicine, Vol. 59, No. 1, January 2018, referred to in the following as Hänscheid et al., discloses a method of estimating absorbed radiation doses from a single late-activity measurement. Neither Jackson nor Hänscheid handle dose uncertainties in a satisfactory way.

Jonathan I. Gear et al.: EANM practical guidance on uncertainty analysis for molecular radiotherapy absorbed dose calculations, European Journal of Nuclear Medicine and Molecular Imaging (2018)45:2456-2474, proposes a framework for modelling uncertainties in the dosimetry chain. The absorbed dose in a site of interest is calculated as the product of the cumulated activity and a dose factor. Each activity value is obtained in terms of a count rate, a calibration factor and a recovery factor, and a correction for partial volume effects. The equation proposed in the article by Gear et al. will not work for a single time-point curve.

Summary of the invention

It is an object of the present disclosure to facilitate dosimetry in radiopharmaceutical therapy, in particular in view of the capacity of equipment and personnel in the clinic.

The present disclosure relates to a method of performing dosimetry for radiopharmaceutical therapy involving the distribution of a radionuclide to a patient, comprising

- obtaining one or more nuclear images of a region of a patient,
- defining a volume of interest in the region,
- determining an activity curve representative of the activity in the volume of interest based on a single nuclear image of the patient taken at a point in time after a treatment fraction using reference data, the activity being representative of the number of radioactive decays as a function of time,
- calculating a total activity within the volume of interest, as a function of time, based on the activity curve
- calculating an uncertainty for the total activity based on the activity curve
- determining a time-integrated activity within the volume of interest based on the total activity.
- calculating an absorbed dose based on the time-integrated activity and calculating an uncertainty in the absorbed dose based on the uncertainty for the total activity, such as image uncertainties.

The volume of interest can be determined in any suitable way, typically based on anatomical images of the patient. The image uncertainties may be caused for example by movement or delineation uncertainties.

The uncertainty may include one or more of the following:

- quantification, including calibration and conversion from counts to activity. This is typically a single scaling factor but may be a 3D representation
- uncertainties relating to how the radionuclides are distributed in regions smaller than the image resolution and/or how they are internalized in the cell r
- image uncertainties relating to patient geometry, such as patient positioning, size, and/or activity in regions where there is movement during the imaging
- image reconstruction dependent uncertainties Each selected reconstruction method will have specific uncertainties and inaccuracy with respect to the ground truth
- recovery correction uncertainty
- segmentation uncertainty, such as uncertainty in the volume and total count uncertainty
- uncertainty in the determined time-activity curve
- dose computation uncertainty

By this method, dosimetry for radiopharmaceutical therapy may be performed based on only a single time-point image of the patient with associated uncertainties. This is advantageous because it reduces the need for hospital resources, including both equipment and staff, and also reduces the strain on the patient.

The uncertainty may be a value for just one of the uncertainties mentioned above, or for any combination of two or more of them. Normally, quantification uncertainties should be considered, possibly in combination with uncertainty in the time-integrated activity and/or segmentation uncertainty. On an overall level, two methods are foreseen for uncertainty estimation: error propagation and robust optimization, respectively.

Which uncertainties to consider in each specific case depends, among other things, on the distance between different regions of interest, compared to the decay range of the radionuclide used, since this will determine how much a change in the activity in one ROI will affect any other ROI. It will also be dependent on the risk of movement of any part of the treatment area, the image quality and other factors.

Error propagation is particularly suitable in the case of a single, well differentiated ROI, with any OAR located far away from any ROI. One way of determining this would be if the activity inside a margin around the delineated ROI is much smaller than the activity inside the ROI. In error propagation, the basic method is for each ROI to estimate all uncertainty components that are to be considered and calculate the sum of these components, using error propagation. The result is a single uncertainty value for each ROI dose value. Such methods may also be used for dose combination with EBRT. For example, three different dose levels may be set for each ROI: minimum, maximum and mean value. This could preferably be handled by considering the sum of the mean absorbed dose and the dose uncertainty when optimizing the EBRT plan. This would reduce the risk of providing a too high dose to the OAR.

The method according to the disclosure may be used in robust optimization of the radionuclide treatment. In this case, the method further comprises determining at least a first and a second activity scenario regarding radionuclide activity and performing robust optimization based on the at least first and second radionuclide scenarios. The method then involves estimating uncertainty components for each ROI and using these components in a robust evaluation of the dose.

The output would be DVHs for the ROIs. This is particularly well suited for situations involving multiple non-differentiated ROIs, meaning that activity in one ROI can lead to dose deposit in one or more other ROIs.

When used in robust optimization of additional external beam radiation treatment, the method further comprises determining at least a first and a second EBRT scenario regarding external beam radiation therapy - EBRT- and performing robust optimization with regard to the first and second EBRT scenario. Different dose levels of the DVHs could be set to compute different scenarios for the ROI combinations.

The method preferably further comprises the steps of obtaining a recovery factor representative of the count rate within the volume of interest, selecting a type of uncertainty and calculating the uncertainty based on the recovery factor.

In preferred embodiments, the method further comprises the step of obtaining a calibration factor representative of the ratio between the count rate and radiation activity within the volume of interest, and calculating the uncertainty based on the calibration factor. The calibration factor can be one factor for the whole body or be differentiated for different parts of the body, or example for different regions of interest.

The reference data may include any of the following:

- cohort data related to the decay of the radionuclide within a number of bodies for a specific treatment scheme, and/or
- previous imaging cycles of the patient and/or
- information regarding the decay of the radionuclide within a body.

The reference data may also be based on a purely single time-point based methodology.

If patient-cohort data are used, a procedure as disclosed in Jackson et al. may be used to estimate the dose. In this case, multiple time point fits, i.e. multiple curves, are used for the patient cohort data. The amplitude of each curve is scaled to match the datapoint available from the single timepoint image, using sums of monoexponential parametrizations of curves. The scaling is used as a weight indicating the magnitude of the change required to adapt the curve to the single time-point. The required change depends on the difference between the value of the multiple time point curve at the time of the single time point and the value of the single time point and may be proportional to the fraction of the two values. In other words, a curve requiring a large change will be assigned a lower weight than a curve requiring a small change. The estimated TIA is calculated as the weighted average of the time-integrated curves from the patient cohort data.

To enable a clinician to take action, the method may further comprise the step of visualizing the absorbed dose and the uncertainty in the absorbed dose.

The disclosure also relates to a computer program product comprising computer readable code means which when run in a computer will cause the computer to perform the steps according to any one of the embodiments above. The computer program product may be stored on a carrier, such as a non-transitory storage medium.

The disclosure also relates to an apparatus for performing dosimetry for radiopharmaceutical therapy, comprising a program memory having stored therein a computer program product according to the above.

Curve-fitting of the time-activity curve is in most cases done with a mono- or multiple-exponential model. In this case, the aim is to estimate or approximate the decay constants $\lambda$ and amplitudes in the functions. The decay $\lambda$ is caused by two factors: the physical decay $\lambda_{\text{phys}}$ of the radionuclide and the biological decay $\lambda_{\text{bio}}$, caused by the washout in the patient's body. The latter is dependent on where in the body the radionuclide is located. The life $\tau$ of the radionuclide within the body is expressed as

$$\tau = 1/\lambda = 1/\lambda_{\text{phys}} + 1/\lambda_{\text{bio}}$$

The disclosure also relates to a radiotherapy planning system comprising a program memory holding such a computer program product and a processor configured to run the computer program product.

Terms and acronyms

- DVH - Dose-Volume Histogram
- OAR - organ at risk. An organ that should be spared from dose as much as possible
- ROI - Region of interest. May be a target or an OAR or any other type of subregion
- EBRT - External Beam Radiation Therapy
- SIRT - Selective Internal Radiation Therapy
- PSMA - Prostate-specific membrane antigen
- PET - Positron Emitting Tomography
- SPECT - Single Photon Emission Computed Tomography

- VOI - Volume Of Interest
- AI - Artificial Intelligence
- MI - Machine Learning
- TIA - Time-Integrated Activity

Brief description of drawings

The invention will be described in more detail in the following, by way of examples and with reference to the appended drawings.

Fig. 1 discloses a graph illustrating the dose uptake activity around a radionuclide as a function of time.
Fig. 2 illustrates the overall method, taking into account the different uncertainties.
Fig. 3 illustrates by example, a method of estimating the time-integrated activity by using the weighted average of the time-integrated activity from patient cohort data
Fig. 4 shows a first embodiment of the overall method using a single post-treatment image.
Fig. 5 depicts a second embodiment of the overall method.
Fig. 6 is a schematic illustration of a computer system in which the methods of this disclosure may be carried out.

Detailed description of embodiments

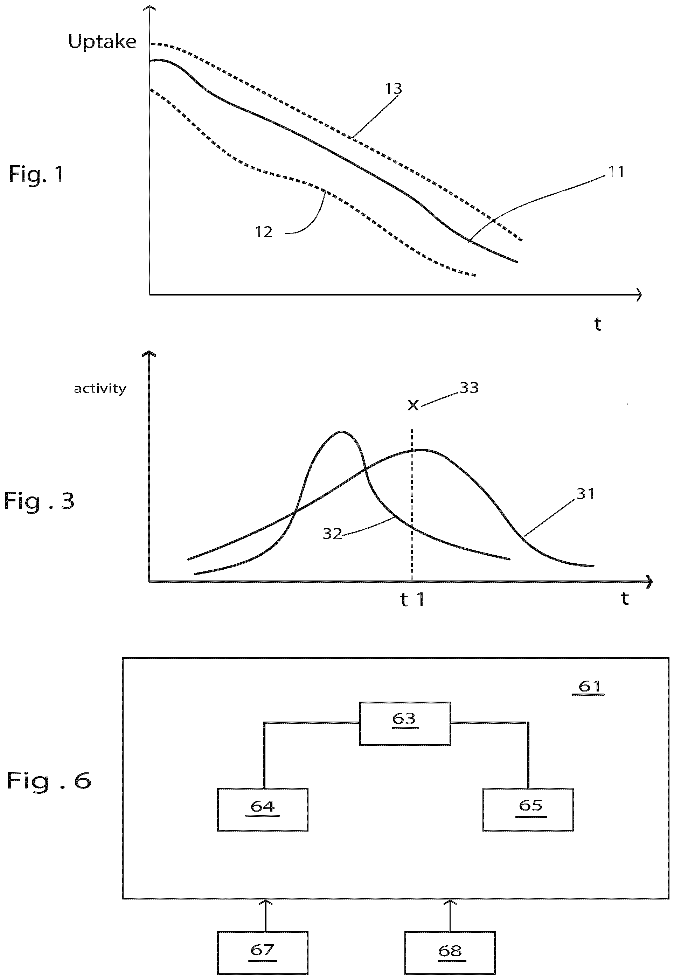

Figure 1 discloses a graph illustrating, as a solid line 11, the dose uptake activity around a radionuclide as a function of time. As can be seen, the activity in this example decreases over time, the accumulated dose being dependent on the area below the curve. In other examples, the activity can also increase with time. The uncertainties in dose uptake also vary with time and are indicated by dashed lines 12, 13 on both sides of the solid line.

In Positron Emitting Tomography (PET) a radioactive tracer is used to visualize and measure changes in various physiological processes within a body. Different tracers are used for different imaging purposes. One field of use is for radiopharmaceutical treatment, where a PET scan can be used to image the activity of the radiopharmaceutical. In these cases, the radiopharmaceutical and the radioactive tracer typically have different properties, leading to differences in pharmacokinetics between the PET tracer and the treatment radionuclide. These differences will lead to uncertainties in the estimated dose.

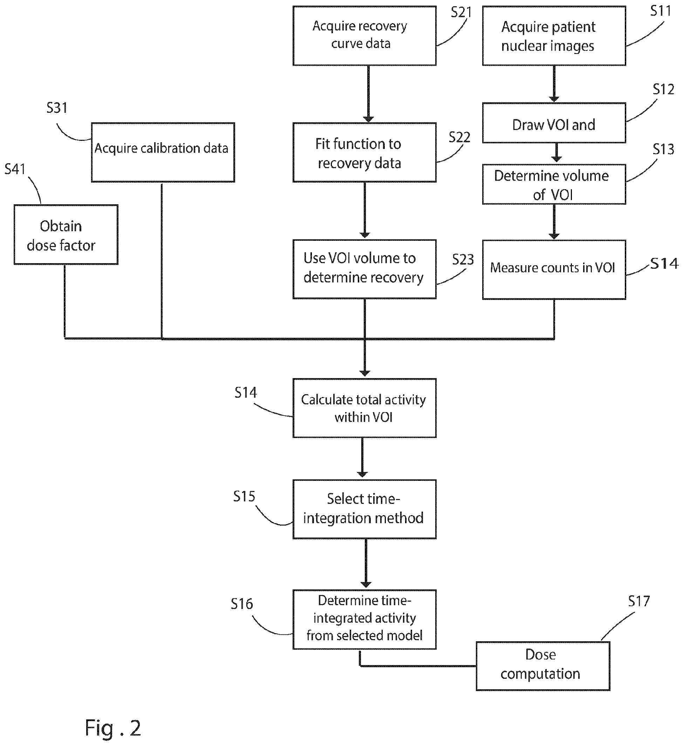

Other factors leading to dose uncertainties will be discussed in connection with Figure 2.

Fig. 2 illustrates an overall method of dosimetry according to the present disclosure, taking into account the different uncertainties and how they affect each other, specifically how they propagate through the procedure.

In a first branch of the method, patient nuclear images are acquired S11, and a volume of interest (VOI) is identified S 12. This may be done in any suitable way, including using artificial intellingence (AI) or machine learning (ML) contouring. Next, its volume is measured S 13. In a following step S14, the counts of events in the volume is measured. For PET images, the events are detections of coinciding photons, whereas for SPECT images a detected photon constitutes an event. Typically, only photons in a chosen energy range will be considered as events. Determining the VOI usually involves outlining the VOI in the scans. In step S13 there will be an uncertainty in the volume, caused by the inaccuracy of the outlining of the VOI. This uncertainty will lead to an uncertainty in the measuring of counts in step S14, since the number of counts is a function of the volume.

In a second branch of the method, recovery curve data are acquired in a first step S21. The recovery curve is a function dependent on the volume, that is, f(V). For each volume Vi, the recovery curve function will return a recovery factor $f(V_i)$. Next, a detector-specific function depending on volume and shape, which can be fitted to a phantom measurement, is fit to the recovery curve data, in step S22, and the VOI volume determined in S 13 is used in step S23 to determine a recovery factor. Generally, the detector-specific function is a function depending on volume, and optionally on shape, which can be fitted to the phantom measurement at hand. It is typically a fixed model, which is determined for each detector, and in the case of SPECT, for the combination of detector and the radionuclide used.

In particular, when iterative image reconstruction is used, the activity in a smaller object will be underestimated. The recovery factor for a particular volume is therefore used to compensate for this, to obtain a value that is closer to the true activity. The recovery curve can be estimated based on phantom measurements, fitting to data points from the recovery curve measurements, and is defined as $R = C/C_{total}$, where C is the observed count rate measured within a VOI matching the true volume of a phantom and $C_{total}$ is the count rate of all counts originating from the phantom.

For a more advanced compensation, a technique not just estimating a curve may be applied to enable taking the shape of an object into consideration, in addition to its volume.

In a third branch, a calibration factor is obtained in step S31. The calibration factor is used to convert the count rate to activity. As mentioned above, this calibration factor may a scaling factor that is the same for the entire image or may vary for different parts of the image. The sensitivity of the system is determined in a manner known in the art by using a source of known activity $A_{cal}$, and measuring the total count rate $C_{ref}$ of $A_{cal}$ under the same conditions as for the current method. The uncertainty in the calibration factor is usually expressed as a fixed percentage of the calibration factor. Methods for determining dose calibration uncertainty are known in the art.

In addition, a dose factor may be used, as indicated by step S41, in a fourth branch. The dose factor represents the relationship between the absorbed dose and the time-integrated activity, and also includes an uncertainty. The dose factor is preferably obtained on a voxel basis from a Monte Carlo dose engine or other dose engine with similar capabilities like a kernel-based dose. Alternatively, the dose factor may be an S-factor obtained based on tabulated values and providing data as a mean value for a whole organ or region. Alternatively, dose can also be assumed to be deposited locally.

In step S14, the total activity within the VOI is calculated based on the measured counts in the VOI from step S14, the recovery factor determined in step S23 and the calibration data acquired in step S31 and optionally the dose factor obtained in step S41.

In step S15, a suitable time-integration method is selected, which will be used to create an activity curve. In step S16, in a first substep, the curve is determined based on the activity calculated in step S14 by fitting the parameters and in a second substep, the time-integrated activity is determined, using the method selected in step S15. In step S17, dose computation is performed based on the time-integrated activity. In subsequent steps, the result of the dose calculation may be visualized in any suitable way. Several ways of visualizing dose are known to the skilled person, including Dose Volume Histograms (DVH), dose maps, average dose and maximum dose.

In addition to the uncertainties shown explicitly in Fig. 2, one or more of the following uncertainties may also be considered:

- quantification including calibration and conversion from counts to activity. This is typically a single scaling factor but may be a 3D representation
- uncertainties relating to how the radionuclides are distributed in regions smaller than the image resolution and/or how they are internalized in the cell r
- image uncertainties relating to patient geometry, such as patient positioning, size, and/or activity in regions where there is movement during the imaging
- image reconstruction dependent uncertainties
- recovery correction uncertainty
- segmentation uncertainty, such as uncertainty in the volume and total count uncertainty
- uncertainty in the determined time-activity curve
- dose computation uncertainty

Two examples of methods that are often used are from Jackson et al. and Hänscheid et al., which are mentioned in the background section. Jackson et al estimate the time-integrated activity by using the weighted average of the time-integrated activity from patient cohort data, as illustrated, somewhat simplified, in Fig. 3. In Fig. 3, a first 31 and a second curve 32, representing activity as a function of time, taken from patient cohort data. Normally, there will be several curves, but only two are shown here for illustration purposes. A point 33 is the single time-point measurement available for the current patient, based on a dosimetry image taken at time t=t1. The curves 31 and 32 are adjusted to the single time point, and weighted in dependence of the adjustment needed. As discussed above, the weight indicating the magnitude of the change required to adapt the curve to the single time-point. The required change depends on the difference between the value of the multiple time point curve at the time of the single time point and the value of the single time point and may be proportional to the fraction of the two values. This means, in the example shown in Fig. 3, that the first curve 31 will be given a higher weight than the second curve 32, because the first curve, at the single time point t1 is closer than the second curve to the single time-point measurement 33.

Hänscheid et al use an approximation of the exponential term for a mono-exponentially integrated activity to estimate the time-integrated activity without the need for an effective half-life:

$$\text{``}\tilde{A} = A(t_1) * \frac{2^{\frac{t_1}{T_{eff}}} * T_{eff}}{\ln(2)} \approx A(t_1) * \frac{2 * t_1}{\ln(2)} \text{ (Eq. 5 and 6)}$$

with imaging time-point $t_1$"

Jackson et al. do not propose a general method for providing uncertainty estimations. Hänscheid et al. discusses how to determine an uncertainty interval depending on the time point of the single measurement. In a single time-point method this can only be an approximation, using a reference value which is in itself associated with an uncertainty. This uncertainty interval can be computed if the true distribution for each OAR is known (i.e. the data is available and fitted). In a pure single timepoint method the true curve is not known, hence, a reference value has to be used, which is available from literature or may be estimated by a systematic study of effective half-lives or literature data. The EANM recommendations mentioned in the background section propose a way of obtaining such estimates for multi-time-point curves but would not work for a single-time-point curve.

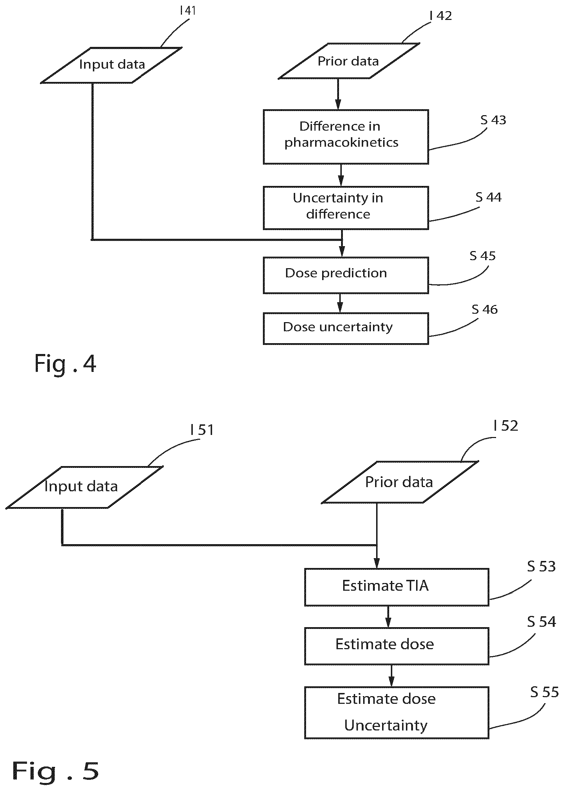

Figure 4 depicts a method according to one embodiment of the disclosure, using a predictive, pre-treatment PET image as input data 141. Based on prior data I42, a difference in pharmacokinetics between the PET tracer and the treatment radiopharmaceutical are determined S43, and the uncertainty in the difference is determined in a subsequent step S44. The prior data may be, for example, patient cohort data or data from images from earlier treatment fractions from the current patient, and the uncertainty is typically determined based on statistical uncertainty. The prior data may also be information about the decay, or half-life, of the radionuclide used in the treatment. As mentioned above, the decay is dependent both on the physical decay of the radionuclide and the biological decay caused by biological functions in the body, such as washout. The biological decay will be dependent on where in the body the radionuclide is located.

If the prior data I42 includes images from earlier treatment fractions, they typically include a pre-treatment PET and one or more treatment SPECT images. If patient cohort data is used as prior data I42, it may be used to estimate the (weighted) average decay parameters for the patient treated using the patient cohort decay curves for the same treatment scheme, where the cohort data includes multiple time-points for the estimation of the decay curves. Alternatively, data from previous treatment fractions of the same patient can be used. For the following discussion, it will be assumed that the prior data includes a pre-treatment PET image and one or more treatment SPECT images.

It may be assumed that the pharmaco-kinetics will be the same over the whole delivery, or an estimate of how pharmaco-kinetics changes over the treatment course may be applied for greater accuracy. In many cases, it can be assumed that the PET-distribution will yield a sufficiently good result. Alternatively, a correlation between the PET and the SPECT distribution could be established. This might involve using a machine learning technique. The training data would include existing PET and SPECT distributions having a known correlation, and the trained model could then be used to predict a SPECT image as output from a PET image used as input data. The uncertainties could then be estimated as the mean standard deviation over multiple patient datasets of PET and SPECT for each organ or in another suitable manner. The difference in pharmacokinetics, is used, in a subsequent step, to predict the dose that will be delivered to the patient in a particular treatment fraction.

In step S45, the resulting dose is predicted and in step S46, the dose uncertainty arising from differences in pharmacokinetics of the PET tracer and the treatment radiopharmaceutical is calculated. This involves either estimating the uncertainty for the PET scan and adding uncertainty considerations for the SPECT scan, in view of decreased spatial resolution and calibration uncertainties.

A second embodiment is illustrated in Figure 5, using a single post-treatment image 151, such as a SPECT/CT or PET/CT image to estimate the treatment dose. Prior data I52 includes patient cohort data or multiple timepoint post-treatment data from a previous treatment fraction, or a pure single timepoint methodology. In the latter case, the uncertainty considered arises from the uncertainty at the post-treatment imaging timepoint, that is, the point in time of taking the post-treatment image. Next, in step S53, the time-integrated activity is estimated according to a suitable method. In step S54, a time-integrated dose is calculated based on the time-integrated activity, and in step S55, the uncertainties in the time-integrated dose are calculated.

One possible method of estimating the time-integrated activity in step S54 is disclosed in Jackson et al. According to this method, the time-integrated activity is estimated by using the weighted average of the time-integrated activity from patient cohort data.

An alternative method for estimating the time-integrated activity in step S54 is proposed by Hänscheid. In this method, the time-integrated activity is estimated. One way of doing this would be using an approximation for the time-integrated activity only dependent on one parameter to estimate the time-integrated activity without the need for an effective half life, e.g. for a mono-exponential case, based on the equation

$$\tilde{A} = A(t_1) * \frac{2^{\frac{t_1}{T_{eff}}} * T_{eff}}{\ln(2)} \approx A(t_1) * \frac{2*t_1}{\ln(2)}$$

In this case, therefore, the aim is to approximate the parameters in the function that will enable modelling of the time-integrated activity.

The information about different uncertainties coming into play during the dose computation can be used to estimate a dose uncertainty and evaluate robustly, e.g. using multiple scenarios to perform a robust evaluation. This would involve defining a number of relevant scenarios. Alternatively, it could involve taking the dose estimation and applying Gaussian error propagation for each step of the dose estimation in what would constitute an extended version of the EANM recommendations but for single time-points. Alternatively, the single uncertainties can be input to a robust dose evaluation for combining radiopharmaceutical therapy and EBRT, to get a robust upper limit for the deliverable dose to an ROI, in particular to an OAR. This might involve considering DVH bands for each uncertainty separately and combined.

Examples of ROIs that are normally well differentiated include parotid glands. In such cases it may be sufficient to consider uncertainties of the ROI concerned, since the influence from activity in other ROIs will be negligible. Parotid glands are OARs in PSMA therapy but normally far from any lesions compared to the relatively short decay range of Lutetium-177. Uncertainties in this case typically include image quantification, delineation uncertainties and quantification uncertainties, including uncertainties in activity integration over time. Movement uncertainties are typically rather small but may be considered.

For an ROI in close proximity of another ROI, compared to the decay range of the radionuclide, but still well differentiated, it may also be sufficient to consider only the uncertainties of the ROI in question. An example of this is a lesion located inside a vertebrae. Although such a lesion will be located close to the bone marrow, which is an OAR, the movement inside the vertebrae, but outside the bone marrow, is small and the range of the nuclide decay products is small. Therefore, the effect on the bone marrow of increased activity in the lesion would normally be small. Uncertainties in this case typically include image quantification, delineation uncertainties and quantification uncertainties, including uncertainties in activity integration over time. As mentioned, movement uncertainties are typically rather small but may be considered.

In the case of overlapping ROIs, activity from one ROI will affect other ROIs, leading to quantification errors and the dose being over- or underestimated. An example of this is a target volume located inside a healthy liver, in SIRT treatment which involves decay products having a rather long range. Uncertainties to consider in this case include image quantification, delineation uncertainties and quantification uncertainties, including uncertainties in activity integration over time, density uncertainty, and movement uncertainties.

Overlapping may also occur because of movement, for example if the liver moves during image acquisition. In addition to the uncertainties mentioned above, the activity from one ROI may be appear in the image to occur in another ROI, which will lead to quantification errors and the dose being over- or underestimated. Also, the movement uncertainties will be rather large. In such cases robust optimization considering different scenarios can give a more realistic estimation of the uncertainty.

For a combination of radionuclide therapy and EBRT, the aim will be to boost the dose in the target volume. In a situation where ROIs are far away from each other compared to the decay range of the radionuclide used, error propagation can be used to determine the dose range in any OAR and the plan can be created to maximize the dose to the target under the constraints of any maximum dose to the OAR.

In the case where activity in one ROI may influence the dose to another ROI, a good strategy would be to create a plan for multiple scenarios (target size, time-integrated activity etc.) and optimize the EBRT plan for there multiple scenarios under the constraints. Plans will look different for different scenarios but one could make sure that the optimization constraints for the OAR are matched for each case. This may lead to a slightly inferior final plan with respect to target dose but a more robust plan with respect to sparing the OAR.

Fig. 6 illustrates schematically a computer system 60 in which one or both methods of the present disclosure may be performed. The computer system may be dose enginge or a dose planning system, or any computer system suitable for performing dose planning. The computer system 60 comprises a computer 61 comprises a processor 63, a data memory 64 and a program memory 65. Preferably, one or more user input means 67, 68 are also present, in the form of a keyboard, a mouse, a joystick, voice recognition means or any other available user input means. The user input means may also be arranged to receive data from an external memory unit.

The data memory 64 comprises input data to be used in the procedures, such as patient images, dose objectives, information about sectors and/or target parts. The data memory may also comprise output data from the procedures. The treatment plan may be generated in the computer 61, or received from another storage means in any way known in the art.

As will be understood, the data memory 64 is only shown schematically. There may be several data memory units, each holding one or more different types of data, for example, one data memory for the treatment plan, one for the patient images, etc. The program memory 65, also only shown schematically, holds a computer program arranged to control the processor to perform one or more of the methods disclosed in this application.

## Claims

1. A method of performing dosimetry for radiopharmaceutical therapy involving the distribution of a radionuclide to a patient, comprising

   - obtaining one or more nuclear images of a region of a patient,
   - defining (S 12) a volume of interest in the region,
   - determining (S 16) an activity curve representative of the activity in the volume of interest based on a single nuclear image of the patient taken at a point in time after a treatment fraction using reference data, the activity being representative of the number of radioactive decays as a function of time,

- calculating (S13, S14) a total activity within the volume of interest, as a function of time, based on the activity curve
- calculating (S44) an uncertainty for the total activity based on the activity curve
- determining (S 16) a time-integrated activity within the volume of interest based on the total activity.
- calculating (S45, S46; S54, S55) an absorbed dose based on the time-integrated activity and an uncertainty in the absorbed dose based on the uncertainty for the total activity, such as image uncertainties.

2. A method according to claim 1, further comprising the step of

- obtaining (S23) a recovery factor representative of the count rate within the volume of interest, selecting an uncertainty and calculating the uncertainty based on the recovery factor.

3. A method according to any one of the preceding claims, further comprising the step of obtaining (S31) a calibration factor representative of the ratio between the count rate and radiation activity within the volume of interest, and calculating the uncertainty based on the calibration factor.

4. A method according to any one of the preceding claims wherein the reference data includes cohort data related to the decay of the radionuclide within a number of bodies.

5. A method according to any one of the preceding claims, wherein the reference data includes previous imaging cycles of the patient.

6. A method according to any one of the preceding claims, wherein the reference data include information regarding the decay of the radionuclide within a body.

7. A method according to any one of the preceding claims, further comprising the step of visualizing the absorbed dose and the uncertainty in the absorbed dose.

8. A method according to any one of the preceding claims, further comprising determining at least a first and a second activity scenario regarding radionuclide activity and performing robust optimization based on the at least first and second radionuclide scenarios.

9. A method according to any one of the preceding claims, further comprising determining at least a first and a second EBRT scenario regarding external beam radiation therapy - EBRT- and performing robust optimization with regard to the first and second EBRT scenario, considering the uncertainty in the absorbed dose from the radionuclide treatment.

10. A computer program product comprising computer readable code means which when run in a computer will cause the computer to perform the steps according to any one of the claims 1 - 8.

11. An apparatus (61) for performing dosimetry for radiopharmaceutical therapy, comprising a program memory (65) having stored therein a computer program product according to claim 9.

Fig. 1

Uptake

13

11

12

t

Fig . 3

activity

33
x

31

32

t 1

t

Fig . 6

61

63

64

65

67

68

Fig . 2

Fig . 4

Fig . 5

Europäisches
Patentamt

European
Patent Office

Office européen
des brevets

# EUROPEAN SEARCH REPORT

**Application Number**

EP 25 15 5478

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | JACKSON PRICE A. ET AL: "Radiation Dosimetry in 177 Lu-PSMA-617 Therapy Using a Single Posttreatment SPECT/CT Scan: A Novel Methodology to Generate Time- and Tissue-Specific Dose Factors", THE JOURNAL OF NUCLEAR MEDICINE, vol. 61, no. 7, 5 December 2019 (2019-12-05), pages 1030-1036, XP093290625, US ISSN: 0161-5505, DOI: 10.2967/jnumed.119.233411 Retrieved from the Internet: URL:https://jnm.snmjournals.org/content/jn umed/61/7/1030.full.pdf> * the whole document * ----- | 1-11 | INV. A61N5/10 |
| Y | SJÖGREEN GLEISNER KATARINA ET AL: "EANM dosimetry committee recommendations for dosimetry of 177Lu-labelled somatostatin-receptor- and PSMA-targeting ligands", EUROPEAN JOURNAL OF NUCLEAR MEDICINE AND MOLECULAR IMAGING, SPRINGER BERLIN HEIDELBERG, BERLIN/HEIDELBERG, vol. 49, no. 6, 14 March 2022 (2022-03-14) , pages 1778-1809, XP037800697, ISSN: 1619-7070, DOI: 10.1007/S00259-022-05727-7 [retrieved on 2022-03-14] * the whole document * ----- -/-- | 1-11 | TECHNICAL FIELDS SEARCHED (IPC) A61N |

The present search report has been drawn up for all claims

| Place of search | Date of completion of the search | Examiner |
|---|---|---|
| Munich | 27 June 2025 | Beck, Ewa |

EPO FORM 1503 03.82 (P04C01)

page 1 of 3

Europäisches Patentamt

European Patent Office

Office européen des brevets

# EUROPEAN SEARCH REPORT

**Application Number**

EP 25 15 5478

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GÖTZ TH I ET AL: "Factors affecting accuracy of S values and determination of time-integrated activity in clinical Lu-177 dosimetry", ANNALS OF NUCLEAR MEDICINE, JAPANESE SOCIETY OF NUCLEAR MEDICINE, TOKYO, JP, vol. 33, no. 7, 22 May 2019 (2019-05-22), pages 521-531, XP036828990, ISSN: 0914-7187, DOI: 10.1007/S12149-019-01365-6 [retrieved on 2019-05-22] * "Results"; page 526 * | 1-11 | |
| Y,D | HÄNSCHEID HERIBERT ET AL: "Dose Mapping After Endoradiotherapy with 177 Lu-DOTATATE/DOTATOC by a Single Measurement After 4 Days", THE JOURNAL OF NUCLEAR MEDICINE, vol. 59, no. 1, 6 June 2017 (2017-06-06), pages 75-81, XP093290626, US ISSN: 0161-5505, DOI: 10.2967/jnumed.117.193706 Retrieved from the Internet: URL:https://jnm.snmjournals.org/content/jn umed/59/1/75.full.pdf> * the whole document * | 1-11 | **TECHNICAL FIELDS SEARCHED (IPC)** |

-/--

The present search report has been drawn up for all claims

| Place of search | Date of completion of the search | Examiner |
|---|---|---|
| Munich | 27 June 2025 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches
Patentamt

European
Patent Office

Office européen
des brevets

## EUROPEAN SEARCH REPORT

**Application Number**

EP 25 15 5478

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DEVASIA THERESA P. ET AL: "A Novel Time-Activity Information-Sharing Approach Using Nonlinear Mixed Models for Patient-Specific Dosimetry with Reduced Imaging Time Points: Application in SPECT/CT After 177 Lu-DOTATATE", THE JOURNAL OF NUCLEAR MEDICINE, vol. 62, no. 8, 18 December 2020 (2020-12-18), pages 1118-1125, XP093249666, US ISSN: 0161-5505, DOI: 10.2967/jnumed.120.256255 * the whole document * | 1-11 | |
| Y | MADSEN MARK T. ET AL: "Technical Note: Single time point dose estimate for exponential clearance", MEDICAL PHYSICS., vol. 45, no. 5, 16 April 2018 (2018-04-16), pages 2318-2324, XP093290690, US ISSN: 0094-2405, DOI: 10.1002/mp.12886 Retrieved from the Internet: URL:https://pmc.ncbi.nlm.nih.gov/articles/PMC5948162/pdf/MP-45-2318.pdf> * the whole document * | 1-11 | TECHNICAL FIELDS SEARCHED (IPC) |
| A | US 2019/168029 A1 (CACHOVAN MICHAL [DE] ET AL) 6 June 2019 (2019-06-06) * paragraph [0038] - paragraph [0044] * | 7 | |

The present search report has been drawn up for all claims

| Place of search | Date of completion of the search | Examiner |
|---|---|---|
| Munich | 27 June 2025 | Beck, Ewa |

EPO FORM 1503 03.82 (P04C01)

## ANNEX TO THE EUROPEAN SEARCH REPORT
## ON EUROPEAN PATENT APPLICATION NO.

EP 25 15 5478

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2025

| Patent document cited in search report | Publication date | Patent family member(s) | Publication date |
|---|---|---|---|
| US 2019168029 A1 | 06-06-2019 | NONE | |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JACKSON et al.** Radiation Dosimetry in Lu-PSMA-617 Therapy Using a Single Posttreatment SPECT/CT Scan: A Novel Methodology to Generate Time- and Tissue-Specific Dose Factors. *The Journal of Nuclear Medicine*, July 2020, vol. 61 (7)
- **HÄNSCHEID et al.** Dose Mapping After Endoradiotherapy with 177Lu-DOTATATE/DOTATOC by a Single Measurement After 4 Days. *The Journal of Nuclear Medicine*, January 2018, vol. 59 (1)

- **JONATHAN I. GEAR et al.** EANM practical guidance on uncertainty analysis for molecular radiotherapy absorbed dose calculations. *European Journal of Nuclear Medicine and Molecular Imaging*, 2018, vol. 45, 2456-2474